# EUROPEAN PATENT APPLICATION

(11) **EP 4 115 837 A1**
(43) Date of publication of application: **11.01.2023**
(21) Application number: 21184666.2
(22) Date of filing: 09.07.2021
(51) Int. Cl.: A61B 34/10

(54) **GUIDANCE IN VENTRICULAR DRAIN PLACEMENT**

(71) Applicant: Koninklijke Philips N.V., 5656 AG Eindhoven (NL)
(72) Inventor: HOLTHUIZEN, Ronaldus Frederik Johannes, 5656 AE Eindhoven (NL); HENDRIKS, Bernardus Hendrikus Wilhelmus, 5656 AE Eindhoven (NL); ELENBAAS, Thijs, 5656 AE Eindhoven (NL); HOMAN, Robert Johannes Frederik, 5656 AE Eindhoven (NL); DRIES, Johan Juliana, 5656 AE Eindhoven (NL)
(74) Representative: Philips Intellectual Property & Standards

(57) **Abstract**

The present invention relates to ventricular drain placement. In order to provide improved guidance in ventricular drain placement for emergency situations, a guiding device (10) for emergency ventricular drain placement is provided comprising an image data input (12), a data processor (14) and an output interface (16). The image data input provides optical sensor-based image data of a head of a subject. The data processor generates an optical shape of the head based on the image data and provides a plurality of datasets of heads with different head shapes, wherein each dataset comprises a spatially assigned trajectory for a ventricular drain placement for the respective head shape. The data processor maps the generated optical shape of the head with at least two of the head shapes of the plurality of datasets and determine a best matching head shape of the dataset and further selects the respective assigned trajectory. The output interface provides a projection of the selected assigned trajectory in spatial relation to the head of the subject as adapted guiding path for ventricular drain placement for the subject based on the mapping of the generated optical shape of the head and the determined best matching head shape.

## Description

### FIELD OF THE INVENTION

The present invention relates to ventricular drain placement, and relates in particular to a guiding device for emergency ventricular drain placement, to a guiding system for emergency ventricular drain placement and to a method for guidance in emergency ventricular drain placement.

### BACKGROUND OF THE INVENTION

Ventricular drain placement is a neurological technique applied e.g. for pressure relief. Besides planned interventions, ventricular drain placement is also used in emergency situations, where speed is a crucial factor. Ventricular drain placement can be performed freehand. For emergency cases, a so-called 'best optimal route' via the Kocher point may be manually determined by the surgeon. For planned interventions, image guidance may be provided. For example, pre-operative CT images of the subject are used and registered to a current situation. As an example, WO 2019/063215 A1 provides medical imaging modalities like CT, X-ray, MRI or ultrasound imaging. However, the lack of pre-operation CT data or workflow issues for providing such medical imaging techniques may occur in emergency situations.

### SUMMARY OF THE INVENTION

There may thus be a need for improved guidance in ventricular drain placement for emergency situations.

The object of the present invention is solved by the subject-matter of the independent claims; further embodiments are incorporated in the dependent claims.

It should be noted that the following described aspects of the invention apply also for the guiding device for emergency ventricular drain placement, for the guiding system for emergency ventricular drain placement and for the method for guidance in emergency ventricular drain placement.

According to the present invention, a guiding device for emergency ventricular drain placement is provided. The guiding device comprises an image data input, a data processor and an output interface. The image data input is configured to provide optical sensor-based image data of a head of a subject. The data processor is configured to generate an optical shape of the head based on the image data. The data processor is also configured to provide a plurality of datasets of heads with different head shapes. Each dataset comprises a spatially assigned trajectory for a ventricular drain placement for the respective head shape. The data processor is further configured to map the generated optical shape of the head with at least two of the head shapes of the plurality of datasets. The data processor is still further configured to determine a best matching head shape of the dataset and to select the respective assigned trajectory. The output interface is configured to provide a projection of the selected assigned trajectory in spatial relation to the head of the subject as adapted guiding path for ventricular drain placement for the subject based on the mapping of the generated optical shape of the head and the determined best matching head shape.

As an advantage, the adapted guiding path for ventricular drain placement can be provided without the need for complex medical imaging like X-ray or MRI. Further, the process does not require any marker on the subject (e.g. patient). Further, the imaging of the subject is provided as a facilitated imaging procedure in form of optical imaging, such as with a camera providing images based on visible light. This allows image acquisition suitable for emergency cases where procedures like X-ray or even ultrasound have proven to be too complex.

As a result, an improved workflow and efficiency of the procedure is provided which is in particular useful for emergency situations.

According to an example, the optical sensor-based image data comprises three-dimensional information about the subject's head.

According to the present invention, a guiding system for emergency ventricular drain placement is provided. The guiding system comprises a guiding device according to one of the preceding examples. The guiding system also comprises an optical sensor device and a display device. The optical sensor device is configured to acquire the optical sensor-based image data of a head of a subject and is configured to transmit or supply this to the data processor via the image data input. The display device is configured to visualize the projection of the selected assigned trajectory in spatial relation to the head of the subject.

According to an example, a user-wearable headset is provided that comprises the optical sensor device and the display device. As an option, the headset is tracked in relation to the subject's head. As a further option, in addition or alternatively, the data processor is configured to perform simultaneous localization and mapping.

According to an example, the display device is configured to provide the projection of the selected assigned trajectory overlaid to reality for a user.

According to an example, the optical sensor device comprises at least one depth camera that provides three-dimensional data of the subject.

According to an example, the wearable headset is a head-mounted display equipped with a camera providing the image data of the head of the subject.

According to an example, the output interface is configured to provide the adapted guiding path for ventricular drain placement for the subject to the user combined with a visualization of at least a part of the head of the subject.

In a first option, the adapted guiding path is provided combined with the visualization as augmented reality, in which the adapted guiding path is overlaid to reality in the user's field of view.

In an alternative or additional second option, the adapted guiding path is provided combined with the visualization as virtual reality, in which the adapted guiding path is overlaid to a projection representing the reality in the user's field of view.

According to an example, the data processor is configured to determine a spatial relation of the user's field of view and the subject's head. The display device is configured to project the adapted guiding path within the user's field of view overlaid to the subject's head based on the mapping of the generated optical shape of the head and the determined best matching head shape.

According to the present invention, also a method for guidance in emergency ventricular drain placement is provided. The method comprises the following steps:
- providing optical sensor-based image data of a head of a subject;
- generating a optical shape of the head based on the image data;
- providing a plurality of datasets of heads with different head shapes, wherein each dataset comprises a spatially assigned trajectory for a ventricular drain placement for the respective head shape;
- mapping the generated optical shape of the head with at least two of the head shapes of the plurality of datasets;
- determining a best matching head shape of the dataset and selecting the respective assigned trajectory; and
- providing a projection of the selected assigned trajectory in spatial relation to the head of the subject as adapted guiding path for ventricular drain placement for the subject based on the mapping of the generated optical shape of the head and the determined best matching head shape.

According to an aspect, optical data of a subject's head is provided and mapped with head structures of a number of exemplary heads for which trajectories (or paths) for emergency ventricular drain placement are provided. By best matching, a selection of one of the examples is made and the respective assigned trajectory is provided as guidance to the user for the current subject.

According to an aspect, a guiding device for emergency ventricular drain placement is provided comprising an image data input, a data processor and an output interface. The image data input provides optical image data of a head of a subject. The data processor generates an optical shape of the head based on the image data and provides a plurality of datasets of heads with different head shapes, wherein each dataset comprises a spatially assigned trajectory for a ventricular drain placement. The data processor maps the generated optical shape of the head with at least two of the head shapes of the plurality of datasets and determines a best matching head shape of the dataset and further selects the respective assigned trajectory. The output interface provides a projection of the selected assigned trajectory in spatial relation to the head of the subject as adapted guiding path for ventricular drain placement based on the mapping of the generated optical shape of the head and the determined best matching head shape.

These and other aspects of the present invention will become apparent from and be elucidated with reference to the embodiments described hereinafter.

### BRIEF DESCRIPTION OF THE DRAWINGS

Exemplary embodiments of the invention will be described in the following with reference to the following drawings:
Fig. 1 shows a schematic setup of an example of a guiding device for emergency ventricular drain placement.
Fig. 2 shows a schematic setup of guiding system for emergency ventricular drain placement.
Fig. 3 shows an example of the guiding system for emergency ventricular drain placement comprising at least one user-wearable headset in the context of an emergency room in a hospital.
Fig. 4 shows basic steps of an example of a method for guidance in emergency ventricular drain placement.

### DETAILED DESCRIPTION OF EMBODIMENTS

Certain embodiments will now be described in greater details with reference to the accompanying drawings. In the following description, like drawing reference numerals are used for like elements, even in different drawings. The matters defined in the description, such as detailed construction and elements, are provided to assist in a comprehensive understanding of the exemplary embodiments. Also, well-known functions or constructions are not described in detail since they would obscure the embodiments with unnecessary detail. Moreover, expressions such as "at least one of', when preceding a list of elements, modify the entire list of elements and do not modify the individual elements of the list.

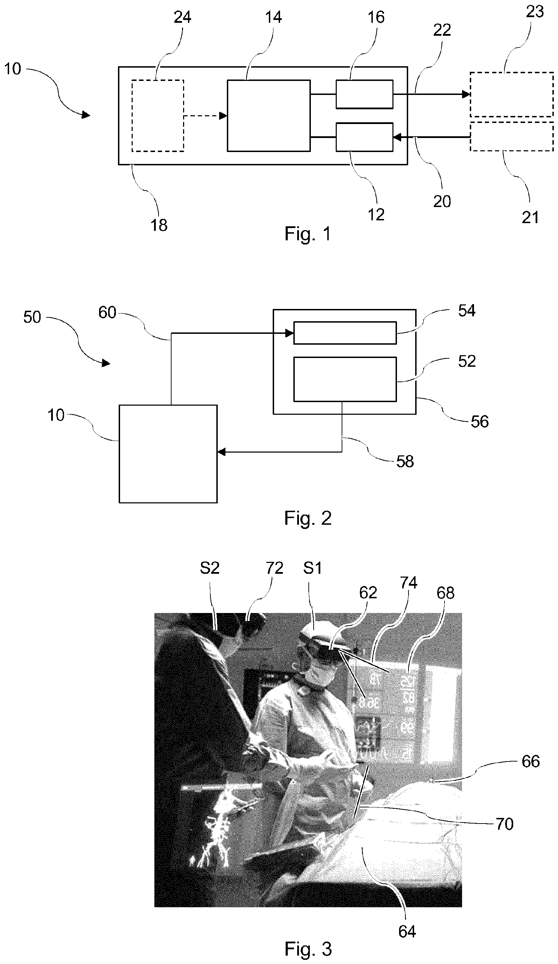

Fig. 1 shows a schematic setup of an example of a guiding device 10 for emergency ventricular drain placement. The device 10 comprises an image data input 12, a data processor 14 and an output interface 16. The image data input 12 is configured to provide optical sensor-based image data of a head of a subject. The data processor 14 is configured to generate an optical shape of the head based on the image data. The data processor 14 is also configured to provide a plurality of datasets of heads with different head shapes. Each dataset comprises a spatially assigned trajectory for a ventricular drain placement for the respective head shape. The data processor 14 is further configured to map the generated optical shape of the head with at least two of the head shapes of the plurality of datasets. The data processor 14 is furthermore configured to determine a best matching head shape of the dataset and to select the respective assigned trajectory. The output interface 16 is configured to provide a projection of the selected assigned trajectory in spatial relation to the head of the subject as adapted guiding path for ventricular drain placement for the subject based on the mapping of the generated optical shape of the head and the determined best matching head shape.

A frame 18 indicates, as an option, the image data input 12, the data processor 14 and the output interface 16 are provided in an integrated manner, such as in a common housing. In another option, not shown, the image data input 12, the data processor 14 and the output interface 16 are provided in a separate manner.

A first data supply arrow 20 indicates the provision of the optical sensor-based image data of the head of a subject, for example as provided by an optial sensor device 21 (indicated as an option with broken lines). A second data supply arrow 22 indicates the provision of the projection of the selected assigned trajectory in spatial relation to the head of the subject as the adapted guiding path for ventricular drain placement for the subject, for example as provided to a display device 23 (also indicated as an option with broken lines).

As an option in Fig. 1, indicated with broken lines, the guiding device 10 comprises a data storage 24 that is configured to store the plurality of (three-dimensional) datasets of heads with different head shapes.

In an example, the optical sensor-based image data is provided by 2D or 3D optical sensors, such as Lidar, structured light sensors, one or more optical cameras and the like. The optical sensor-based image data can also be provided as visual camera-based image data.

Briefly said, optical sensors such as cameras or others indicated above are relied on for the determination of the (outer) shape of the subject's head. Any inside structure is not provided by the supplied image data itself. However, this type of information, being essential for the determination of the trajectory or path running through a part of the head, i.e. bone (skull) and tissue (brain and others), but missing in the current inmage data, is so-to-speak replaced by referring to a plurality of exemplary head structures for which such a trajectory for emergency ventricular drain placement has been pre-determined. The trajectories of the examples already take into account such respective information of inside the head, i.e. those aspects that are not visible by a camera imaging the outer surface of the head. By providing the plurality of examples, the mapping of the head's camera image with the examples and the determining of the best match, the respective current situation inside the subject's head is taken into account for achieving an improved accuracy of the trajectory proposal provided to the user, compared to a pure manual and intuitive emergency ventricular drain placement.

The use of a pre-existing database gives improved personalized emergency ventricular drain path planning and safe zone indication without the need for pre-operative imaging. This enables an improved outcome and efficiency by providing better path planning and trajectory navigation even when pre-operative CT data or ultrasound is not available.

The image data input 12 can also be referred to as image supply, as image data supply, as image input, as input unit or simply as input. In an example, the image data input 12 is data-connectable to an imaging source arrangement like an optical camera providing the optical sensor-based image data, e.g. visual camera-based image data of the subject. In an example, the image data input 12 is actually data-connected to an imaging source like a camera. In an example, the image data input 12 is data-connectable to a data storage having stored the visual camera-based image data.

The data processor 14 can also be referred to as data processing arrangement, as processor unit or as processor. In an example, the data processor 14 is data-connected to the image data input 12 and the output interface 16. In an example, the data processor 14 is provided as mapping or registration engine that matches the current images with stored image data to identify an optimal path or trajectory for emergency ventricular drain placement. For example, the data processor 14 is configured to generate a three-dimensional optical shape of the head. For example, the data processor 14 is configured to provide a plurality of three-dimensional datasets of heads with different head shapes.

The output interface 16 can also be referred to as output or output unit. In an example, the output interface 16 is data-connectable to a display arrangement or display device. In another example, the output interface 16 is data-connected to a display.

Based on a mapping with a database of e.g. previous subjects or patients, the most likely and most suitable optical placement path is calculated and provided as guidance. In an option, augmented reality or optical stereotactic navigation is used to place the ventricular drain along this path.

In another example, a 3D visualization of the target anatomy is shown and the user chooses a path directly by the AR visualization of this target anatomy of the patient, i.e. the subject. By building a database of head shapes, ventricle locations, and areas to avoid, a better, more accurate and more personalized treatment approach can be created than is now possible with the current freehand practice by utilizing the correlation between skull shape and brain location/shape.

The term "to provide optical sensor-based image data of a head of a subject" relates to supplying, transmitting or forwarding the data for further processing steps. The optical sensor-based image data relates to image data based on detection e.g. by a camera sensitive to the visible light spectrum. In an option, thermal radiation is detected by the camera, like infrared light.

The term "to generate an optical shape of the head based on the image data" relates to computing the shape of the head based on the optical images. The optical shape is thus representing the actual physical shape of the head. The generation of the optical shape thus results in three-dimensional information of the head.

The term "to provide a plurality of datasets of heads with different head shapes" relates to supplying, transmitting or forwarding the datasets for further processing steps. The datasets each provide information about a respective head with a certain shape and size and internal structures related that are related to outside detectable form. The plurality comprises at least two different datasets. For example, more than 5, 10, 50 or even 100 datasets are provided, or more than 1000.

As an option, other parameters are provided to augment the selection of the dataset of the target anatomy with the suggested path. For example, parameters like age or sex or other classification aspects are provided.

As an additional optional feature, these parameters are estimated based on the optical images and AI to avoid manually entering said parameters by the user.

The term "spatially assigned trajectory" relates to a trajectory that has been determined for that particular head represented by the dataset. The trajectory is a path information for achieving, e.g. placing, a ventricular drain placement. The trajectory is provided in spatial relation to the respective head data, such as by an entry point, angular arrangement and length, or start and end point, in respect to e.g. a head or body reference system or grid.

The term "to map the generated optical shape of the head with at least two of the head shapes of the plurality of datasets" relates to registering the optical shape of the actual head with the heads shapes of the database. The mapping can be provided as a rigid registration or as an elastic registration. The mapping forms a plurality of pairs of the actual head with a respective one of the heads of the dataset.

The term "to determine a best matching head shape of the dataset" relates to identifying the head shape of the dataset that appears most common with the actual, i.e. current, situation. The best matching step can be provided as a weighted matching step in which predetermined parts are weighted more and others less in respect of the matching. The best matching can also be provided as an overall match of the complete shape.

The term "to select the respective assigned trajectory" relates to taking, i.e. using, the trajectory that belongs to the determined head shape of the dataset.

The term "to provide a projection of the selected assigned trajectory in spatial relation to the head of the subject as adapted guiding path for ventricular drain placement for the subject based on the mapping of the generated optical shape of the head and the determined best matching head shape" relates to presenting the projection of the selected assigned trajectory to the user, e.g. forwarding the projection for further presenting or displaying steps, or showing, i.e. displaying the trajectory.

The optical sensor-based image data can also be provided as visual camera based imaga data.

In an option, the optical sensor-based image data comprises three-dimensional information about the subject's head.

In an example, for the mapping the data processor 14 is configured to compute a facial recognition algorithm. The facial recognition algorithm allows for identifying landmarks and the parts between the landmarks. In an option, a display is provided that is configured to provide the projection of the selected assigned trajectory, i.e. to display the selected assigned trajectory in relation to the subject's head as guidance information. In addition to facial recognition, algorithms that perform simultaneous localization and mapping (SLAM) enable the reconstruction of a more complete 3D head shape determination by observing the subject from multiple angles.

Fig. 2 shows a schematic setup of guiding system 50 for emergency ventricular drain placement. The system 50 comprises a guiding device 10 according to one of the examples above. The system 50 further comprises an optical sensor device 52 and a display device 54. The optical sensor device 52 is configured to acquire the optical sensor-based image data of a head of a subject and is configured to supply this to the data processor 14 via the image data input 12. The display device 54 is configured to visualize the projection of the selected assigned trajectory in spatial relation to the head of the subject.

A frame 56 indicates that, as an option, the optical sensor device 52 and the display device 54 can be provided in an integrated manner, such as in a common structure, e.g. housing. In another option, optical sensor device 52 and the display device 54 are provided in a separate manner.

In an example, the optical sensor device 52, like a camera device, acquires the optical sensor-based image data, like visual camera-based image data.

In an example, the display device 54 visualizes the projection of the selected assigned trajectory.

Fig. 3 shows an example of the guiding system 50 for emergency ventricular drain placement comprising at least one user-wearable headset 62 in the context of an emergency room in a hospital.

As an option, the user-wearable headset 62 comprises the optical sensor device 52 and the display device 54, As an option, the headset 62 is tracked in relation to the subject's head. A first staff member S1 is standing next to a subject 64 (partly covered with drapes) with the subject's head 66 shown in the background. The headset 62 provides information to the user by a respective presentation 68 overlaid to reality within the user's field of view. An interventional device 70 is shown, representing a tool for ventricular drain placement. The displayed projection of the selected assigned trajectory in spatial relation to the head of the subject as the adapted guiding path for ventricular drain placement for the subject provides guidance information to the user for performing the task of ventricular drain placement, in particular in emergency situations when time is essential and further image data of the inside of the subject's head is not readily available.

As an example, Fig. 3 shows the first staff member S1 wearing the user-wearable headset 62. A further staff member S2 is shown, also wearing a headset 72 which can be provided similar manner as the user-wearable headset 62 comprising the optical sensor device 52 and the display device 54.

The user-wearable headset is also referred to as head-mounted gear. In an example, a standard wearable headset technology is provided, like the Microsoft HoloLens (trademarks). An optical shape of the head is constructed using structure light and computer vision.

In an option, the optical sensor is provided as a separate optical sensor, e.g. a separate camera and the spatial relation of the camera, i.e. optical sensor, to the user and the subject is tracked, respectively determined.

The cameras of the headset, such as Microsoft HoloLens, for example, can be used to measure the shape and position of the face of the subject's head. In Fig. 3, this is indicated with an angle 74. This enables tracking of the subject, i.e. the subject's head, without the attachment of optimal markers to the subject. Thus, a marker-free or markerless registration is provided.

The optically measured shape of the head has distinct features that allows the registration of three-dimensional image data of the database to an optical navigation system. By using the combination of the depth sensor, optical cameras and facial recognition algorithms, this registration can be achieved without the need of a touch sensor or manually operated optical laser devices and the like.

In an example, the display device, like the head mounted gear, is configured to provide the projection of the selected assigned trajectory overlaid to reality for a user.

In an example (not shown in detail), the optical sensor device comprises at least one depth camera that provides three-dimensional data of the subject.

The use of a depth camera, for example, allows to track the subject position and even to register the subject without markers or touching of the subject.

By using the depth camera, the head shape of the subject can be measured and a better and more personalized emergency ventricular drain trajectory can be selected for the subject.

In an option, the camera is a time-of-flight camera.

The camera provides only image data of the outer surface of the subject, such as the visible outer parts. This allows a quick and facilitated image acquisition. The image acquisition is thus X-ray free, and also free of any other subject-, i.e. tissue-penetrating imaging like X-ray or ultrasound. The connection with the samples of the database provides for certain simplification, but allows a fast assignment of a trajectory for the emergency ventricular drain placement. In an example, the database thus provides improved and more accurate guidance.

To register optional pre-operative image data of the database to the navigation system, a registration algorithm is used that maps the skin visible in the dataset volume to the position and shape of the head. The usage of a depth camera plus optical cameras plus a facial recognition algorithm is provided.

In an option, next to the shape of the head, other parameters can also be used such as age, gender, known diseases etc. to improve the estimation of the brain and ventricle shape. A deep learning method can be used to calculate to optical correlation of the trajectory (and safe zone) to the subject without the need of personalized pre-op CT imaging. It is also suitable for acute drainage procedure in case of life-threatening brain haemorrhage, for example, if the target area of the brain is known to a certain accuracy. As an advantage, the most save predicted passage zone can be determined on the spot.

To apply the determined treatment plan, i.e. trajectory, different techniques are provided. As an example, an augmented reality guidance is shown to the user and the user aligns and places the ventricular drain along the planned path.

In another example, the position of the device is tracked, and three-dimensional optical stereotactic navigation is used.

As an example, the wearable headset is a head-mounted display equipped with a camera providing the image data of the head of the subject.

As another example, the output interface is configured to provide the adapted guiding path for ventricular drain placement for the subject to the user combined with a visualization of at least a part of the head of the subject as one of the group of:
i) augmented reality, wherein the adapted guiding path is overlaid to reality in the user's field of view; or
ii) virtual reality, wherein the adapted guiding path is overlaid to a projection representing the reality in the user's field of view.

As another example, the data processor 14 is configured to determine a spatial relation of the user's field of view and the subject's head. The display device is configured to project the adapted guiding path within the user's field of view overlaid to the subject's head based on the mapping of the generated optical shape of the head and the determined best matching head shape.

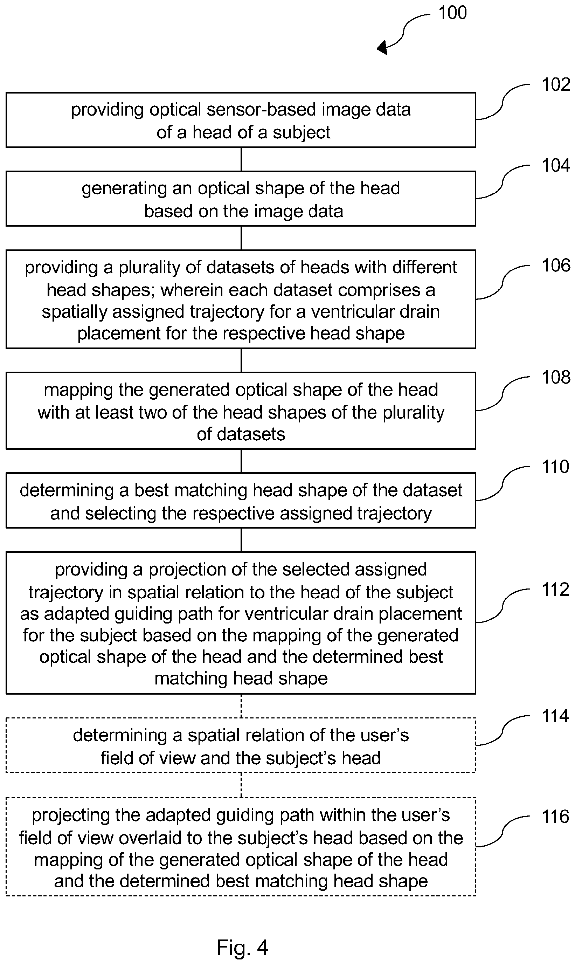

Fig. 4 shows basic steps of an example of a method 100 for guidance in emergency ventricular drain placement. The method comprises the following steps:
- In a first step 102, optical sensor-based image data of a head of a subject is provided.
- In a second step 104, an optical shape of the head is generated based on the image data.
- In a third step 106, a plurality of datasets of heads with different head shapes is provided. Each dataset comprises a spatially assigned trajectory for a ventricular drain placement for the respective head shape.
- In a fourth step 108, the generated optical shape of the head is mapped with at least two of the head shapes of the plurality of datasets.
- In a fifth step 110, a best matching head shape of the dataset is determined and the respective assigned trajectory is selected.
- In a sixth step 112, a projection of the selected assigned trajectory is provided in spatial relation to the head of the subject as adapted guiding path for ventricular drain placement for the subject based on the mapping of the generated optical shape of the head and the determined best matching head shape.

In an example, the optical shape of the head is provided as three-dimensional optical shape of the head. In another example, the optical shape of the head is provided as two-dimensional optical shape of the head.

In an example, the plurality of datasets of heads with different head shapes is provided as three-dimensional datasets. In another example, the plurality of datasets of heads with different head shapes is provided as two-dimensional datasets.

The usage of a database in which the relationship between head shapes and the optimal trajectory to place the emergency ventricular drain placement is established, allows an improved guidance, as for example compared to a one-size fits all trajectory that is used for emergency ventricular drain placement without pre-operation data, such as when applying the so-called Kocher point approach. The Kocher point is a certain entry point on the subject's skull that has been proven to be most suitable for manual placement of the emergency ventricular drain.

In an example of the method, not shown in detail, for the mapping, a sub-step of facial recognition is provided.

In an example of the method, also not shown in detail, the optical sensor-based image data of the head of the subject is provided by a user wearable headset. In an option of the method, the wearable headset is a head-mounted display equipped with a camera providing the image data of the head of the subject.

In an example of the method, not shown in detail, the adapted guiding path for ventricular drain placement for the subject is provided to the user combined with a visualization of at least a part of the head of the subject as one of the group of:
i) augmented reality, wherein the adapted guiding path is overlaid to reality in the user's field of view; or
ii) virtual reality, wherein the adapted guiding path is overlaid to a projection representing the reality in the user's field of view.

In another example, the adapted guiding path is provided to the user as augmented virtuality wherein the adapted guiding path is combined with an image of an interventional device for providing the ventricular drain hold by the user, which image is overlaid to a projection of the determined dataset of the plurality of datasets of heads.

Fig. 4 indicates two further steps as an option, illustrated with broken lines:
- In a first additional step 114, a spatial relation of the user's field of view and the subject's head is determined.
- In a second additional step 116, the adapted guiding path is projected within the user's field of view overlaid to the subject's head based on the mapping of the generated optical shape of the head and the determined best matching head shape.

In an example, not further shown in detail, a computer program is provided enabling a processor to carry out the method of one of the examples above.

In an example, a computer program or program element for controlling an apparatus according to one of the examples above is provided, which program or program element, when being executed by a processing unit, is adapted to perform the method steps of one of the method examples above.

In an example, not further shown in detail, a computer readable medium is provided having stored the program element of claim of the examples above.

The term "subject" may also be referred to as individual. The "subject" may further also be referred to as patient, although it is noted that this term does not indicate whether any illness or disease is actually present with the subject.

In another exemplary embodiment of the present invention, a computer program or a computer program element is provided that is characterized by being adapted to execute the method steps of the method according to one of the preceding embodiments, on an appropriate system.

The computer program element might therefore be stored on a computer unit or be distributed over more than one computer units, which might also be part of an embodiment of the present invention. This computing unit may be adapted to perform or induce a performing of the steps of the method described above. Moreover, it may be adapted to operate the components of the above described apparatus. The computing unit can be adapted to operate automatically and/or to execute the orders of a user. A computer program may be loaded into a working memory of a data processor. The data processor may thus be equipped to carry out the method of the invention.

Aspects of the invention may be implemented in a computer program product, which may be a collection of computer program instructions stored on a computer readable storage device which may be executed by a computer. The instructions of the present invention may be in any interpretable or executable code mechanism, including but not limited to scripts, interpretable programs, dynamic link libraries (DLLs) or Java classes. The instructions can be provided as complete executable programs, partial executable programs, as modifications to existing programs (e.g. updates) or extensions for existing programs (e.g. plugins). Moreover, parts of the processing of the present invention may be distributed over multiple computers or processors.

As discussed above, the processing unit, for instance a controller implements the control method. The controller can be implemented in numerous ways, with software and/or hardware, to perform the various functions required. A processor is one example of a controller which employs one or more microprocessors that may be programmed using software (e.g., microcode) to perform the required functions. A controller may however be implemented with or without employing a processor, and also may be implemented as a combination of dedicated hardware to perform some functions and a processor (e.g., one or more programmed microprocessors and associated circuitry) to perform other functions.

Examples of controller components that may be employed in various embodiments of the present disclosure include, but are not limited to, conventional microprocessors, application specific integrated circuits (ASICs), and field-programmable gate arrays (FPGAs).

This exemplary embodiment of the invention covers both, a computer program that right from the beginning uses the invention and a computer program that by means of an up-date turns an existing program into a program that uses the invention.

Further on, the computer program element might be able to provide all necessary steps to fulfil the procedure of an exemplary embodiment of the method as described above.

According to a further exemplary embodiment of the present invention, a computer readable medium, such as a CD-ROM, is presented wherein the computer readable medium has a computer program element stored on it which computer program element is described by the preceding section. A computer program may be stored and/or distributed on a suitable medium, such as an optical storage medium or a solid-state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated, and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfil the functions of several items re-cited in the claims. The mere fact that certain measures are re-cited in mutually different dependent claims does not indicate that a combination of these measures cannot be used to advantage. Any reference signs in the claims should not be construed as limiting the scope.

## Claims

1. A guiding device (10) for emergency ventricular drain placement, the guiding device comprising:
- an image data input (12);
- a data processor (14); and
- an output interface (16);
wherein the image data input is configured to provide optical sensor-based image data of a head of a subject;
wherein the data processor is configured to generate an optical shape of the head based on the image data; to provide a plurality of datasets of heads with different head shapes, wherein each dataset comprises a spatially assigned trajectory for a ventricular drain placement for the respective head shape; to map the generated optical shape of the head with at least two of the head shapes of the plurality of datasets; to determine a best matching head shape of the dataset and to select the respective assigned trajectory; and
wherein the output interface is configured to provide a projection of the selected assigned trajectory in spatial relation to the head of the subject as adapted guiding path for ventricular drain placement for the subject based on the mapping of the generated optical shape of the head and the determined best matching head shape.

2. Device according to claim 1, wherein the optical sensor-based image data comprises three-dimensional information about the subject's head.

3. Device according to claim 1 or 2, wherein the guiding device comprises a data storage (24) that is configured to store the plurality of (three-dimensional) datasets of heads with different head shapes.

4. Device according to claim 1, 2 or 3, wherein, for the mapping, the data processor is configured to compute a facial recognition algorithm.

5. A guiding system (50) for emergency ventricular drain placement, the guiding system comprising:
- a guiding device (10) according to one of the preceding claims;
- an optical sensor device (52); and
- a display device (54);
wherein the optical sensor device is configured to acquire the optical sensor-based image data of a head of a subject and is configured to supply this to the data processor via the image data input; and
wherein the display device is configured to visualize the projection of the selected assigned trajectory in spatial relation to the head of the subject.

6. System according to claim 5, wherein a user-wearable headset (62) is provided that comprises the optical sensor device and the display device;
wherein the headset is tracked in relation to the subject's head; and wherein the data processor is configured to perform simultaneous localization and mapping.

7. System according to claim 5 or 6, wherein the display device is configured to provide the projection of the selected assigned trajectory overlaid to reality for a user.

8. System according to claim 5, 6 or 7, wherein the optical sensor device comprises at least one depth camera that provides three-dimensional data of the subject.

9. System according to one of the claims 6 to 8, wherein the wearable headset is a head-mounted display equipped with a camera providing the image data of the head of the subject.

10. System according to one of the claims 5 to 9, wherein the output interface is configured to provide the adapted guiding path for ventricular drain placement for the subject to the user combined with a visualization of at least a part of the head of the subject as one of the group of:
i) augmented reality, wherein the adapted guiding path is overlaid to reality in the user's field of view; or
ii) virtual reality, wherein the adapted guiding path is overlaid to a projection representing the reality in the user's field of view.

11. System according to one of the claims 5 to 10, wherein the data processor is configured to determine a spatial relation of the user's field of view and the subject's head; and
wherein the display device is configured to project the adapted guiding path within the user's field of view overlaid to the subject's head based on the mapping of the generated optical shape of the head and the determined best matching head shape.

12. A method (100) for guidance in emergency ventricular drain placement, the method comprising the following steps:
- providing (102) optical sensor-based image data of a head of a subject;
- generating (104) an optical shape of the head based on the image data;
- providing (106) a plurality of datasets of heads with different head shapes; wherein each dataset comprises a spatially assigned trajectory for a ventricular drain placement for the respective head shape;
- mapping (108) the generated optical shape of the head with at least two of the head shapes of the plurality of datasets;
- determining (110) a best matching head shape of the dataset and selecting the respective assigned trajectory; and
- providing (112) a projection of the selected assigned trajectory in spatial relation to the head of the subject as adapted guiding path for ventricular drain placement for the subject based on the mapping of the generated optical shape of the head and the determined best matching head shape.

13. Method according to claim 12, wherein a spatial relation of the user's field of view and the subject's head is determined (114); and
wherein the adapted guiding path is projected (116) within the user's field of view overlaid to the subject's head based on the mapping of the generated optical shape of the head and the determined best matching head shape.

14. A computer program enabling a processor to carry out the method of claim 12 or 13.

15. A computer readable medium having stored the program element of claim 14.
